Europäisches Patentamt

European Patent Office

Office européen des brevets

(19)

(11) Publication number: **0 454 931 A1**

## EUROPEAN PATENT APPLICATION

(12)

(21) Application number: 90401179.8

(22) Date of filing: 30.04.90

(51) Int. Cl.5: **A61B 7/04**

(43) Date of publication of application:
06.11.91 Bulletin 91/45

(84) Designated Contracting States:
AT BE CH DE DK ES FR GB GR IT LI NL SE

(71) Applicant: **Shue, Ming-Jeng**
No. 14, Lane 8 Chung-I St.
Taichung City(TW)

(72) Inventor: **Shue, Ming-Jeng**
No. 14, Lane 8 Chung-I St.
Taichung City(TW)

(74) Representative: **Bouju, André**
Cabinet André Bouju B.P. 6250
F-75818 Paris Cédex 17(FR)

(54) Electronic stethoscopic apparatus.

(57) An electronic stethoscopic apparatus includes a control housing body 10 adapted for hand gripping and operational control, a signal amplifier 14 and a radio wave transmitter 14' installed inside the control housing body 10, a rotatable shaft 20, transducers 23, 23' disposed inside the rotatable shaft 20 and electrically connected to the signal amplifier 14, at least one chest piece 40 selectively and acoustically coupled to the transducers 23, 23' and mounted to the rotatable shaft 20, and a binaural headpiece 30 electrically connected to the signal amplifier 14. Each chest piece 40 has a concaved disc member 400 and a diaphragm 41 attached to the disc member 400. The diaphragm 41 has a central outward projection 410 with a flat contact face to be placed in direct contact with the patient during auscultation. The binaural headpiece 30 includes a pair of telescopic ducts 31, a pair of ear-phone pieces 32 attached to the telescopic ducts 31, and a pair of conducting wires 33 electrically connecting the ear-phone pieces 32 to the signal amplifier 14. The binaural headpiece 30 can be folded and retracted to make the same compact.

FIG.2

EP 0 454 931 A1

The invention relates to a stethoscope, more particularly to a multi-functional radio/wire stethoscopic apparatus.

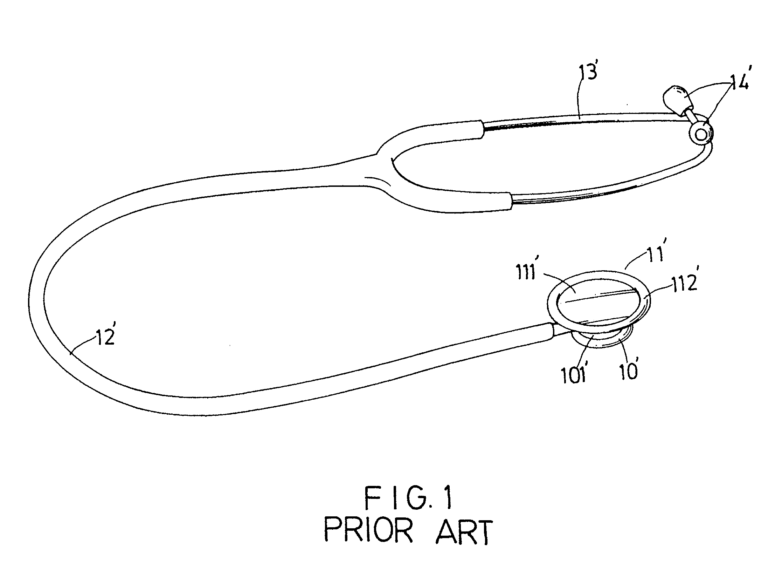

Referring to Figure 1, a conventional auscultatory stethoscope is shown to comprise: a low frequency chest piece 10' (usually an open bell or Ford chest piece); a high frequency chest piece 11' (usually a closed diaphragm or Bowles chest piece); a tubing 12' acoustically coupled to the chest pieces 10', 11'; a binaural headset 13' connected to a forking end of the tubing 12'; and a pair of eartips 14' attached to an extreme end of the binaural headset 13' opposite the tubing 12'. Some of the main features and drawbacks of the above mentioned stethoscope are as follows:

(1) The low frequency chest piece 10' has a peripheral ring edge 101' made of a soft material and is used to auscultate low pitched body sounds such as third and fourth heart sounds, and most murmurs. When placed in direct contact with a patient's skin, the ring edge 101' confines a tightly sealed air compartment with the skin. Airtight sealing is necessary in this case to be able to clearly hear the desired body sounds. Thus, if the contact surface is not flat and smooth due to the presence of clothing and the like, auscultation of low pitched body sounds using the chest piece 10' cannot be achieved.

(2) The high frequency chest piece 11' has a flat diaphragm 111'and is used to auscultate high pitched body sounds such as first and second heart sounds, ejection sounds and clicks, the opening snap of mitral or tricuspid stenosis, murmurs of aortic or pulmonary valve regurgitation, and interventricular septal defects. When using the chest piece 11', the diaphragm 111' is tightly pressed against the patient's skin. The soft body tissue directly beneath the diaphragm 111' experiences tension. High frequency vibrations coming from the pulsating movement of the viscus to be examined is transmitted to the body surface. The chest piece 11' has a protruded ring base 112' made of metal, rubber, or plastic, threaded to an upper end of the chest piece 11' for fixing the diaphragm 111' to the same. During auscultation, a fraction of the transmitted vibrations to the diaphragm 111' is damped and absorbed by the ring base 112', thereby resulting in the unreliability of the received signals.

(3) The binaural headset 13' of the conventional stethoscope is usually formed as a rigid body having a fixed and unadjustable length. The binaural headset 13' also takes up a lot of storage space and is bulky because of its size and shape. Furthermore, the binaural headset 13' may be uncomfortable to a user since the length and the positioning of the eartips 14' cannot be adjusted according to the user's need.

Therefore, an object of this invention is to provide an electronic stethoscopic apparatus with a chest piece of a relatively high sensitivity to permit auscultation of low pitched body sounds even if the contact surface is not flat due to the presence of clothing and the like.

A second object of this invention is to provide an electronic stethoscopic apparatus with a chest piece having a base with a plateau-shaped resonance membrane mounted thereon for achieving better body contact as well as better resolution of the body sounds being detected.

A third object of this invention is to provide an electronic stethoscopic apparatus which can precisely detect the noise of internal organs and clearly amplify it for ease of enabling diagnosis.

A fourth object of this invention is to provide an electronic stethoscopic apparatus which includes a radio wave transmitting means built therein for transmitting the sound produced by the body portions of the patient as a radio signal to be received by a remote receiver so as to allow simultaneous auscultation for facilitating consultative diagnosis or for teaching purposes.

Another object of this invention is to provide an electronic stethoscopic apparatus with a resonance membrane which is made of plastic polymers, has a thermal set rim for connecting with the base of the chest piece, and has no protruding ring base which is conventionally used for fixing the diaphragm to the chest piece, thus minimizing absorption and damping losses of vibrations experienced by the diaphragm.

A further object of this invention is to provide an electronic stethoscopic apparatus which has two to three detachably engaged chest pieces of different frequency responses to permit auscultation of patients of varying age, sex and state of health without using a second stethoscope.

Still another object of this invention is to provide an electronic stethoscopic apparatus having a binaural headset with folded telescopic ducts and a pair of ear-phone pieces connected to extreme ends of the telescopic ducts to permit adjustment of the length and the position of the ear-phone pieces according to the needs of the user.

Still a further object of this invention is to provide an electronic stethoscopic apparatus having a binaural headset which can be folded and retracted about a longitudinal axis to make the headset compact and thus save on storage space.

Accordingly, an electronic stethoscopic apparatus of this invention comprises a control housing body adapted for hand gripping and operational control, a signal amplifying means and a radio wave transmitting means installed inside the control housing body, a rotatable shaft means, at least one transducer means disposed inside the rotatable shaft means and electrically connected to the signal amplifying means and radio wave transmitting means, at least one chest piece selectively and acoustically coupled to the transducer means and mounted to the rotatable shaft means, and a binaural headpiece electrically connected to the signal amplifying means.

Each chest piece has a concaved disc member and a diaphragm attached to the disc member. The diaphragm is substantially plateau-shaped and has a hollow central outward projection with a flat contact face to be placed in direct contact with the patient during auscultation. The height and the diameter of the central outward projection varies for each chest piece to correspondingly vary the frequency response.

The binaural headpiece includes a pair of telescopic ducts, a pair of ear-phone pieces attached to the telescopic ducts, and a pair of conducting wires electrically connecting the ear-phone pieces to the signal amplifying means. The binaural headpiece further includes a pair of flexible connectors having sleeve portions respectively sleeved onto the telescopic ducts. The connectors are rotatably hinged to one another so that the binaural headpiece can be folded to make the same compact. The telescopic ducts comprise at least two telescopically connected rigid sections. The telescopic ducts are thus retractable for conveniently carrying the binaural headpiece.

Other features and advantages of this invention will become apparent in the following detailed description of the preferred embodiment with reference to the accompanying drawings, in which:

Figure 1 is an illustration of a conventional stethoscopic apparatus;

Figure 2 is an exploded view of the preferred embodiment of an electronic stethoscopic apparatus according to this invention;

Figure 3 is an exploded view of a portion of the preferred embodiment according to this invention;

Figure 4 is a sectional view of the assembly of Figure 3;

Figures 5, 6 are sectional views of a partially assembled electronic stethoscopic apparatus according to this invention;

Figure 7 is a schematic circuit block diagram of the electrical circuitry of the preferred embodiment;

Figures 8, 9, 10 illustrate a binaural headpiece of the electronic stethoscopic apparatus of this invention; and

Figures 8A, 8B illustrate enlarged portions of the binaural headpiece of Figure 8.

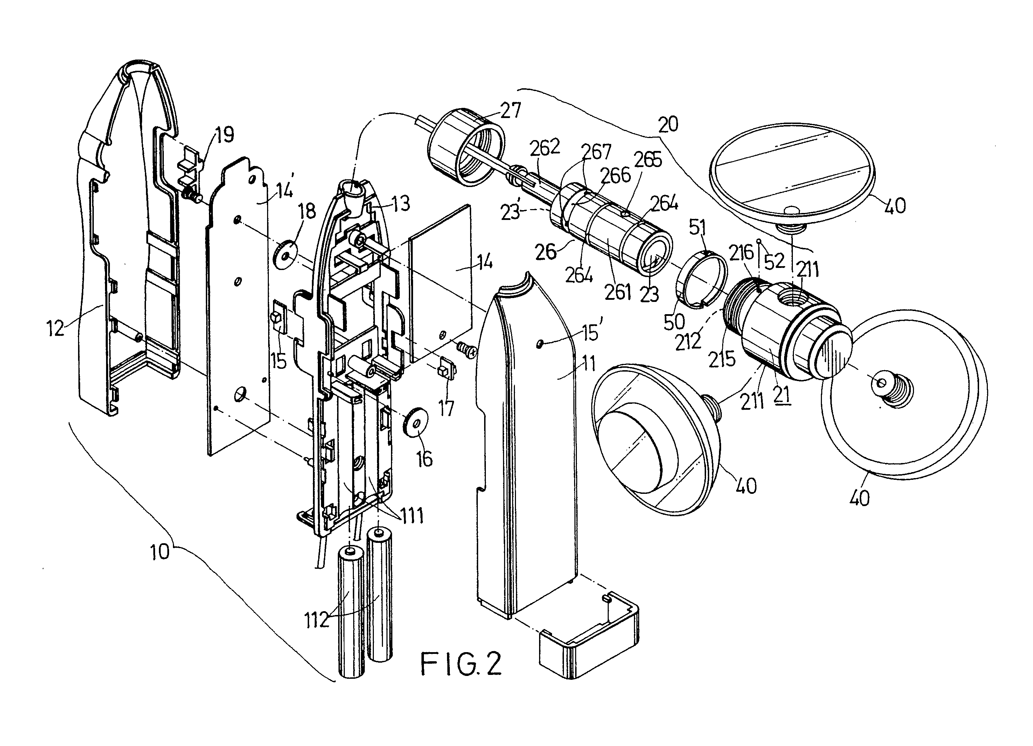

Referring to Figures 2 to 5, the preferred embodiment of an electronic stethoscopic apparatus according to this invention comprises a control housing body 10, a rotatable shaft means 20 and three chest pieces 40 mounted to the rotatable shaft means 20.

The control housing body 10 is adapted for hand-gripping and comprises a front casing 11, a rear casing 12 and a central plate 13 fixed between the front and rear casings 11, 12. A signal amplifying means 14 and a wireless radio wave transmitting means 14' are fixed to opposite sides of the central plate 13. The front casing 11 cooperates with the rear casing 12 to confine a cell compartment 111 positioned below the signal amplifying means 14 for receiving battery cells 112. The control housing body 10 further comprises a power supply and frequency band select switch 15, a tuning means 16, a transmitter switch 17, a volume control means 18, a tact switch 19 and a power supply indicator 15'. The power supply indicator 15' lights when the power supply switch 15 is in an ON position. The intensity of light output of the power supply indicator 15' gives an indication of the remaining power of the battery cells 112.

The rotatable shaft means 20 comprises a hollow chest piece holder 21, a hollow cylindrical centerpiece 22, a pair of transducers 23, 23' facing one another at opposite ends of the cylindrical centerpiece 22, and a hollow cylindrical casing shaft 26. The chest piece holder 21 can be made of metal or plastic and has three angularly spaced mounting holes 211 formed on an upper cylindrical wall of the same. The chest pieces 40 are mounted to the chest piece holder 21 at the mounting holes 211. The chest piece holder 21 has a central hollow space 212 for receiving the cylindrical centerpiece 22 and the cylindrical casing shaft 26. The chest piece holder 21 further has a lower cylindrical wall with a threaded end 215 and ball receiving holes 216 formed above the threaded end 215.

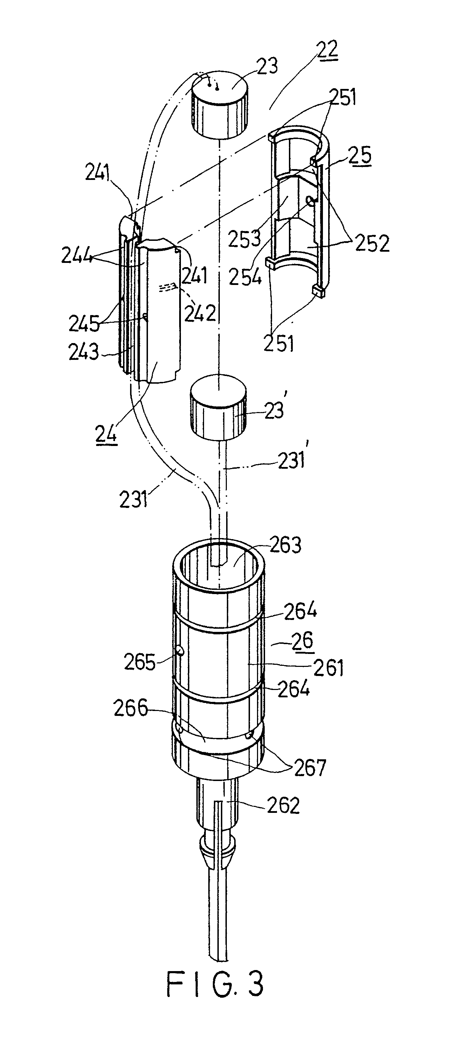

Referring again to Figure 3, the centerpiece 22 is formed as longitudinal halves 24, 25 of a cylindrical wall and is received inside the cylindrical casing shaft 26. The first and second halves 24, 25 are preferably made of rubber or plastic. The first half 24 has corner grooves 241 and the second half 25 has corner protrusions 251 received by the corner grooves 241. The corner grooves 241 and the corner protrusions 251 make it easier to fix the two halves 24, 25 together when mounting to form the cylindrical centerpiece 22. The second half 25 has a pair of transducer receiving spaces 252 on opposite ends. The transducer receiving spaces 252 are separated by an inwardly projecting central section 253 formed with a sound hole 254. The first half 24 has receiving spaces 242 substantially similar to the receiving spaces 252 of the second half 25. The first half 24 does not have a sound hole similar to the sound hole 254, but it has a first axial groove 243 and at least one second axial groove 244 which serves as a path for sound waves. A portion of a conducting wire 231 of the transducer 23 is disposed inside the first axial groove 243 when the transducers 23, 23' are received by the receiving spaces 242, 252.

The cylindrical casing shaft 26, which is made of a rigid material (preferably metal or plastic), has a first cylindrical portion 261. A hollow space 263 confined by the first cylindrical portion 261 receives the cylindrical centerpiece 22 and the transducers 23, 23'. A second cylindrical portion 262 has a diameter substantially smaller than the first cylindrical portion 261 and extends downwards from the same. The second cylindrical portion 262 is fixed to a top end of the control housing body 10. The conducting wires 231, 231' of the transducers 23, 23' pass through the second cylindrical portion 262 so that they can be electrically connected to and serve as inputs to the electrical circuitry of the signal amplifier circuit board 14.

Referring once more to Figure 3, the first cylindrical portion 261 has a pair of first annular grooves 264 and a through hole 265 formed midway between the first annular grooves 264. The first annular grooves 264 contain lubricant to enable ease of rotation of the chest piece holder 21 relative to the cylindrical casing shaft 26 when the cylindrical casing shaft 26 is received by the chest piece holder 21. Thus, the through hole 265 of the cylindrical casing shaft 26 can be communicated with one of the mounting holes 211 of the chest piece holder 21 to allow sound waves coming from the chest piece 40 to pass through the mounting holes 211, the through hole 265, and the sound hole 254 of the cylindrical centerpiece 22 to achieve acoustic coupling of the chest piece 40 with the transducers 23, 23'. The first cylindrical portion 261 further includes a second annular groove 266 disposed near an end adjacent to the second cylindrical portion 262. Through holes 267 are formed at the bottom of the second annular groove 266. When the cylindrical casing shaft 26 is received by the chest piece holder 21, the through holes 267 are in alignment with the ball receiving holes 216 of the chest piece holder 21.

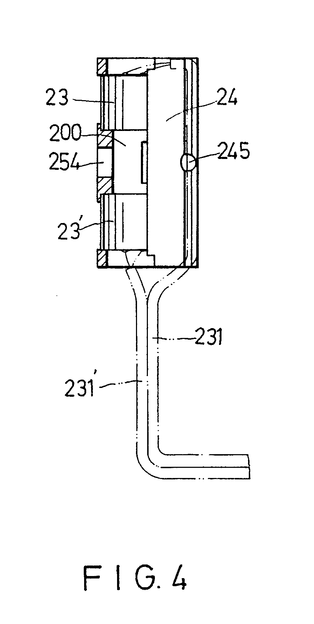

Referring to Figures 2, 4, after the transducers 23, 23' have been mounted inside the cylindrical centerpiece 22, a sound chamber 200 is confined between the transducers 23, 23' and adjacent to the inwardly projecting central section 253. A base cover 27 is fixedly sleeved to the threaded end 215 of the chest piece holder 21 to enclose the cylindrical casing shaft 26 inside the hollow space 212 of the chest piece holder 21. The chest piece holder 21 is rotatable with respect to the cylindrical casing shaft 26 but the cylindrical centerpiece 22 is stationary relative to the cylindrical casing shaft 26 to prevent damage to the conducting wire 231 of the transducer 23 disposed inside the first axial groove 243.

Referring again to Figure 3, each of the second axial grooves 244 of the first half 24 is formed with a central sound outlet hole 245. The second axial grooves 244 and the sound outlet holes 245 serve as physical adjusting means for varying the sound pressure level inside the sound chamber 200 according to the sensitivities of the transducers 23, 23'.

If the transducers 23, 23' used have relatively high sensitivities, the presence of excessive sound pressure inside the sound chamber 200 might cause a build-up of standing waves therein and can result in the over saturation of the transducers 23, 23'. The second axial grooves 244 and the sound outlet holes 245 thus provide a passage for sound waves trapped inside the sound chamber 200 to prevent the build-up of standing waves.

If the transducers 23, 23' used have relatively low sensitivities, the sound pressure level inside the sound chamber 200 must be maintained at a relatively high level. The presence of the sound outlet holes 245 are not necessary and should be blocked.

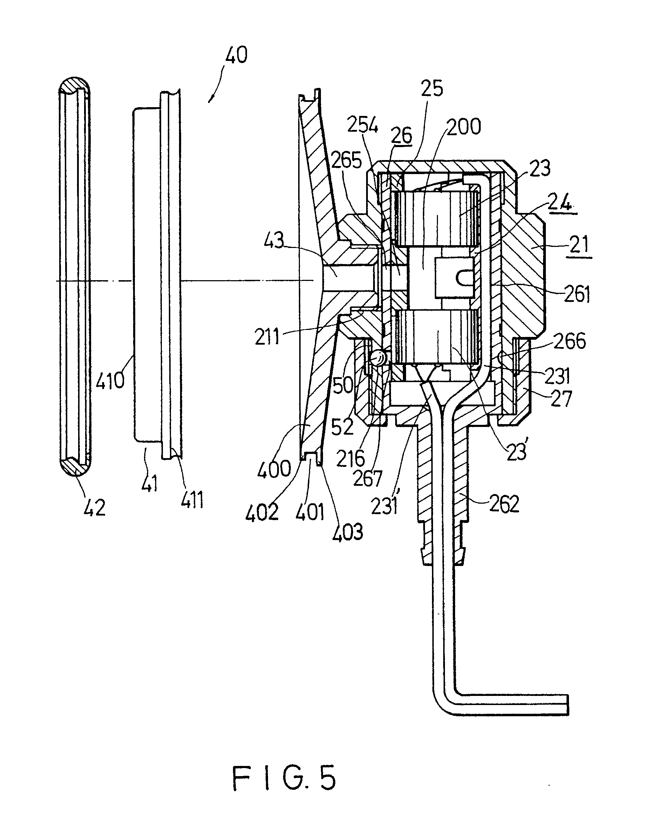

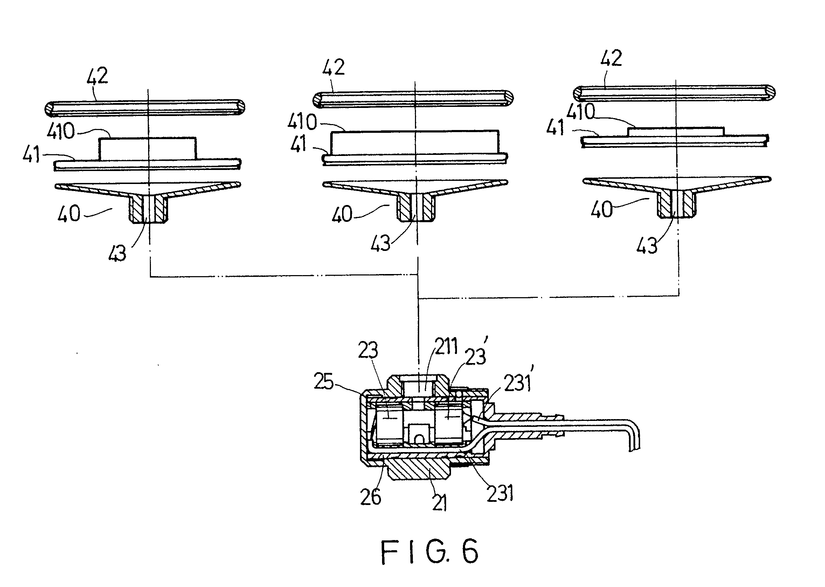

The chest pieces 40 used in the electronic stethoscopic apparatus according to this invention are shown in greater detail in Figures 5, 6. Each chest piece 40 has a concaved disc 400 and a diaphragm or resonance membrane 41 at the periphery of the disc 400. The diaphragms 41 are substantially plateau-shaped and have hollow central outward projections 410 with different heights to vary their corresponding frequency responses. The height of the central outward projection 410 may range from 1.2 millimeters (mm) to 9.6 mm and the diameter thereof may range from 3.2 mm to 40 mm. The diaphragm 41 is preferably made of a thermosetting plastic material. The diaphragm 41 further has a peripheral ring 411. A pair of concentric annular flange portions 402, 403 formed at the periphery of the disc 400 confine an annular groove 401. The diaphragm 41 covers the annular groove 401 and the flange portion 402 of the disc 400. A rubber sealing ring 42 is provided around and engaged with the peripheral ring 411 of the diaphragm 41 and the flange portion 403 of the disc 400. The sealing ring 42 provides airtight sealing between the diaphragm 41 and the disc 400 and prevents abrasion of the two bodies.

During auscultation, the central outward projection 410 of the diaphragm 41 has a flat contact face which is slightly pressed against the chest of the patient. The presence of the central outward projection 410 eliminates the need for applying a strong pressing force against the patient's chest which is usually the case when using a conventional chest piece 10' as shown in Figure 1. The mechanical vibrations experienced by the central outward projection 410 are converted into sound waves that traverse a sound wave duct 43. Since the chest piece 40 does not have a rigid periphery similar to the rigid periphery 112' of the conventional chest piece 10' which is placed in direct contact with the patient's chest, the mechanical vibrations of the diaphragm 41 experience minimal losses due to damping and absorption. The chest pieces 40 of the electronic stethoscopic apparatus according to this invention have relatively high sensitivities and are extremely useful for differential diagnosis. Since the preferred embodiment employs chest pieces 40 of different frequency responses, auscultation of patients of different age, sex, and state of health is possible without using a second stethoscope. The power supply and frequency band switch 15 must be placed, however, in its proper position when changing the chest piece 40 in use from a high frequency chest piece to a low frequency chest piece, or vice versa.

As shown in Figures 2, 5, the preferred embodiment further comprises a C-shaped slice 50 fitted to the lower cylindrical wall of the chest piece holder 21. The C-shaped slice 50 has holes 51 which are aligned with the ball receiving holes 216 of the chest piece holder 21. Engaging steel balls 52 are received by the ball receiving holes 216 of the chest piece holder 21 and protrude into the holes 267 of the cylindrical casing shaft 26 and the holes 51 of the C-shaped slice 50. The C-shaped slice 50 and the engaging steel balls 52 permit rotation of the chest piece holder 21 with respect to the cylindrical casing shaft 26 and resiliently hold the chest piece holder 21 in a releasable engaging position.

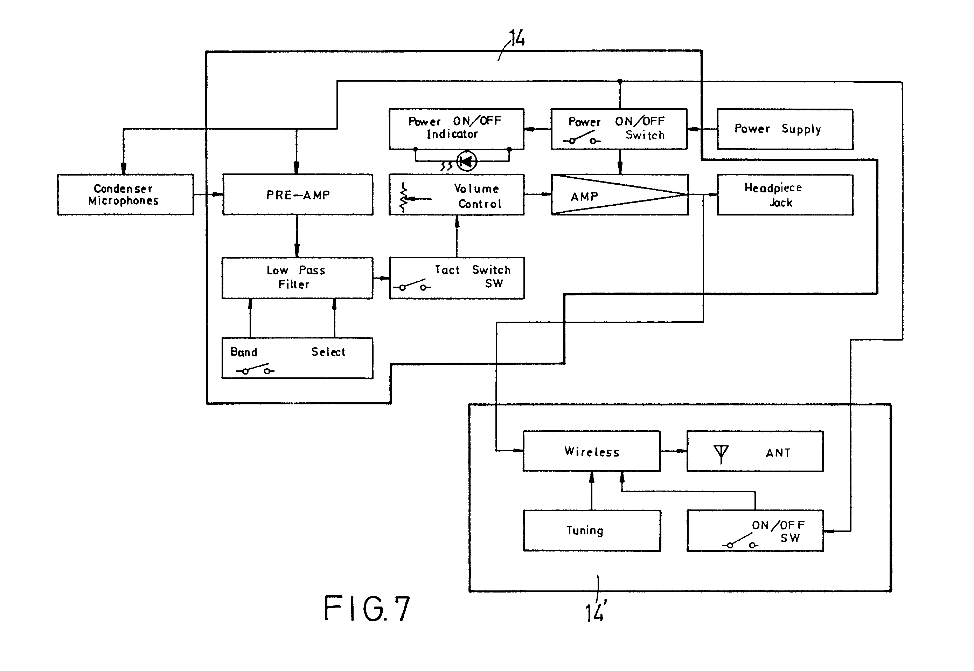

A schematic circuit block diagram of the electrical circuitries of the signal amplifying means 14 and the wireless radio wave transmitting means 14' is shown in Figure 7. The electrical circuitry of this invention is generally similar to the electrical circuit of a conventional electronic stethoscopic apparatus but differs in the addition of the tact switch 19. When the power supply switch 15 is switched ON to actuate the transducers 23, 23', the chest piece 40 is pressed against the chest of the patient before switching the tact switch 19 to a closed position. A filtered electrical signal output of the transducers 23, 23' is then received at the headpiece jack. When auscultation has been accomplished or when it is desired to temporarily stop auscultation, the tact switch 19 is switched to an open position. The filtered electrical signal output of the transducers 23, 23' is disconnected from the amplifier circuit block and no electrical signal can thus be received at the headpiece jack.

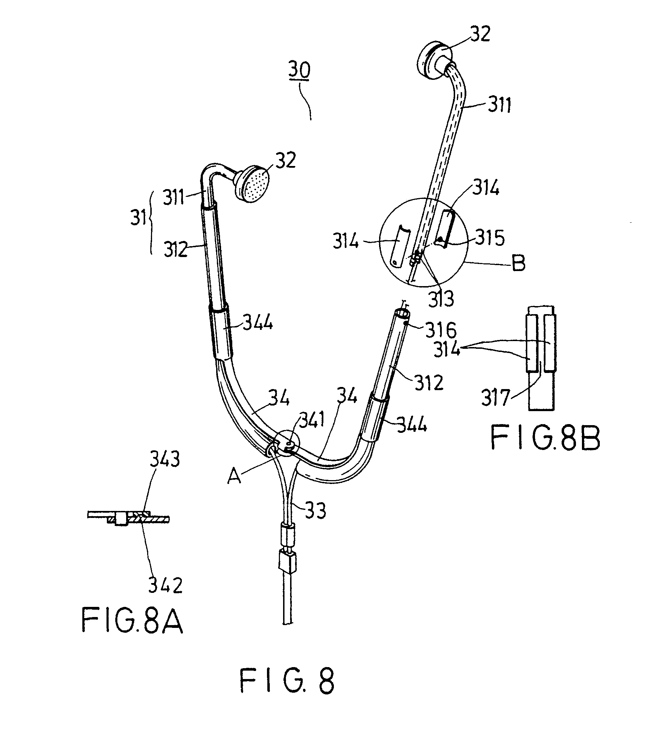

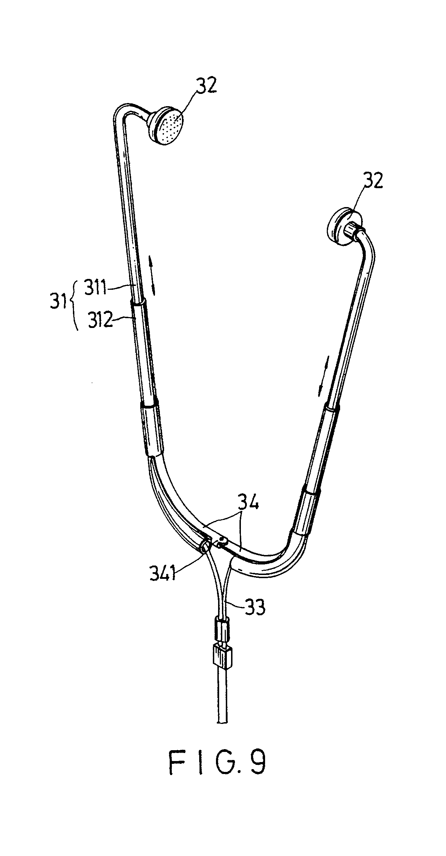

A binaural headpiece 30 of the preferred embodiment is shown in Figures 8, 8A, 8B to comprise a pair of telescopic ducts 31, a pair of earphone pieces 32, and a pair of conducting wires 33 which connect the ear-phone pieces 32 to the electrical circuitry. Each telescopic duct 31 includes a first tube 311 slidably inserted to a substantially L-shaped second tube 312. The earphone pieces 32 are connected to extreme upper ends of the first tubes 311. A pair of diametrically opposed holes 313 are formed on extreme lower ends of the same. A pair of concaving rectangular strips 314 are disposed on opposite sides of each first tube 311 and have protrusions 315 engaged in the holes 313 of the first tube 311. The second tubes 312 have a pair of inwardly projecting stubs 316. During the assembly of the first tube 311 and the second tube 312, the stubs 316 are aligned with and pass through axial grooves 317 formed between the rectangular strips 314. The stubs 316 prevent the first tube 311 from disengaging from the second tube 312 by engaging the rectangular strips 314. The first tubes 311 are thus telescopically mounted to and rotatable relative to the second tube 312, as shown in Figure 9.

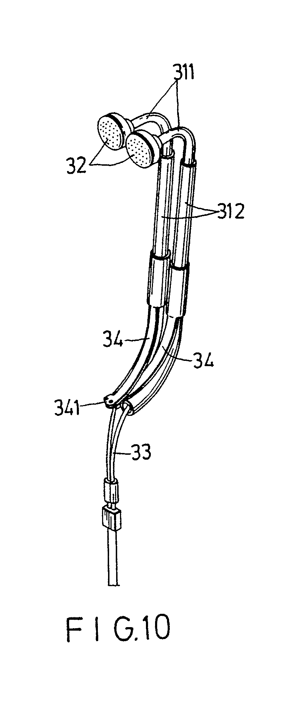

The binaural headpiece 30 further comprises a pair of flexible connectors 34 having hollow sleeve portions 344 respectively sleeved onto the second tubes 312 near curving parts of the same. The connectors 34 are rotatably hinged together at their ends by a hinge pin 341. The second tubes 312 can therefore be folded and retracted about a longitudinal axis of the binaural headpiece 30, as shown in Figure 10, to make the binaural headpiece 30 compact and thus save space during storage. The connectors 34 are provided with a protrusion 342 and a groove 343, respectively, so as to releasably engage with one another to hold the second tubes 312 in position when unfolded.

The main features of the preferred embodiment are as follows:

(A) The structure of the diaphragm 41 is neither concave, convex nor flat. The provision of the central outward projection makes it unnecessary to apply a strong pressing force against the patient's chest. Since the chest pieces 40 do not have any rigid periphery placed in direct contact with the patient's chest, the mechanical vibrations of the diaphragm 41 experience minimal losses due to absorption and damping. The chest pieces 40 also have relatively high sensitivities which are extremely useful for differential diagnosis.

(B) Since the preferred embodiment uses a combination of high and low frequency chest pieces 40, auscultation of patients of varying age, sex, and state of health is possible without using a second stethoscope.

(C) The cylindrical centerpiece 22 of the rotatable shaft means 20 can be made to have axial grooves 244 with sound outlet holes 245 to serve as physical adjusting means for varying the sound pressure level inside the sound chamber 200 according to the sensitivities of the transducers 23, 23' used.

(D) The chest piece holder 21 can be rotated relative to the cylindrical casing shaft 26. The C-shaped slice 50 and the engaging balls 52 resiliently hold the chest piece holder 21 in a releasable engaging position.

(E) The addition of the tact switch 19 to the electrical circuitry allows the disconnection of the transducers 23, 23' from the electrical circuitry when auscultation has been accomplished or when it is desired to temporarily stop auscultation.

(F) The binaural headpiece 30 has a pair of telescopic ducts 31 which can be folded and retracted about a longitudinal axis to make the binaural headpiece 30 compact and thus save on storage space.

## Claims

1. An electronic stethoscopic apparatus comprising a rotatable shaft means (20), transducer means (23, 23') disposed inside said rotatable shaft means (20), at least one chest piece (40) selectively and acoustically coupled to said transducer means (23, 23') and mounted to said rotatable shaft means (20), characterized in that

   said chest piece (40) has a concaved disc member (400) with a periphery (401) and a diaphragm (41) attached to and covering said disc member (400), said diaphragm being substantially plateau-shaped and having a hollow central outward projection (410) with a flat contact face to be placed in direct contact with the patient during auscultation.

2. An electronic stethoscopic apparatus as claimed in Claim 1, wherein said diaphragm (41) has a peripheral ring member (411) sealed, in an airtight relationship, with said periphery (401) of said disc member (400).

3. An electronic stethoscopic apparatus as claimed in Claim 1, wherein said central outward projection (400) has a height ranging from 1.2 mm to 9.6 mm.

4. An electronic stethoscopic apparatus as claimed in Claim 1, wherein said central outward projection has a diameter ranging from 3.2 mm to 40 mm.

5. An electronic stethoscopic apparatus comprising:

   a hollow chest piece holder (21) having an upper cylindrical wall with angularly spaced mounting holes (211);

   a plurality of chest pieces (40) mounted to said chest holder (21) at said mounting holes (211), each of said chest pieces (40) having a substantially plateau-shaped diaphragm (41) with a hollow central outward projection (410) which has a flat contact face to be placed in direct contact with the patient during auscultation;

   a cylindrical casing shaft (26) having a first cylindrical portion (261) and a second cylindrical portion (262) with a restricted cross-section, said first cylindrical portion (261) being received by said chest piece holder (21) and having a sound hole (265), said chest piece holder (21) being rotatable relative to said cylindrical casing shaft (26) to selectively align said sound hole (265) with one of said mounting holes (211);

   a stationary cylindrical centerpiece (22) provided in said cylindrical casing shaft (26) and having a first and a second half of a cylindrical wall (24, 25) confining receiving spaces (242, 252) an a sound chamber (200) between said receiving spaces (242, 252), said second half of a cylindrical wall (25) having a sound hole (254) communicated with said sound chamber (200) and said sound hole (265); and

   two transducer means (23, 23') installed in said receiving spaces (252).

6. An electronic stethoscopic apparatus as claimed in Claim 5, wherein said chest piece holder (21) has a lower cylindrical wall with steel ball receiving holes (216) formed therein; said rotatable shaft means (20) further comprising a C-shaped slice (50) disposed around said lower cylindrical wall of said chest piece holder (21) and surrounding said steel ball receiving holes (216), a cover (27) fixedly sleeved on said lower cylindrical wall of said chest piece holder (21) surrounding said C-shaped slice (50), and an engaging steel ball (52) received in each of said steel ball receiving holes (216) and biased by said C-shaped slice (50) to a releasable engaging position with said cylindrical casing shaft (26).

7. An electronic stethoscopic apparatus as claimed in Claim 5, wherein said first half of a cylindrical wall (24) of said cylindrical centerpiece (22) further has an outer surface with a first axial groove (243) adjacent to an inner surface of said cylindrical casing shaft (26); said electronic stethoscopic apparatus further comprising conducting wires (231, 231') extending through said second cylindrical portion (262) and respectively connected to said transducer means (23, 23'), one of said conducting wires (231, 231') passing through said first axial groove (243).

8. An electronic stethoscopic apparatus as claimed in Claim 5, wherein said first half of a cylindrical wall (24) of said cylindrical centerpiece (22) further having at least one second axial groove (244) angularly spaced from said first axial groove (243) and having a sound outlet hole (245) formed therein; said second axial groove (244) and said sound outlet hole (245) serving as physical adjusting means for varying the sound pressure level inside said sound chamber (200) to prevent the over saturation of the transducers (23, 23').

9. An electronic stethoscopic apparatus as claimed in Claim 1, further comprising:
   a control housing body (10) adapted for hand gripping and operational control;
   a signal amplifying means (14) and a radio wave transmitting means (14') installed inside said control housing body (10) and electrically connected to said transducer means (23, 23'); and
   a binaural headpiece (30) electrically connected to said signal amplifying means (14), said binaural headpiece (30) including a pair of telescopic ducts (31) with extreme upper ends, a pair of ear-phone pieces (32) attached to said extreme upper ends of said telescopic ducts (31), and a pair of conducting wires (33) electrically connecting said ear-phone pieces (32) to said signal amplifying means (14).

10. An electronic stethoscopic apparatus as claimed in Claim 9, wherein each of said telescopic ducts (31) include a first tube (311) slidably inserted to a second tube (312); said binaural headpiece (30) further comprises a pair of flexible connectors (34) having sleeve portions (344) respectively sleeved onto said telescopic ducts (31), said connectors (34) being rotatably hinged to one another;
   whereby, said connectors (34) can be folded and said telescopic ducts (31) can be retracted to make said binaural headpiece (30) compact.

11. An electronic stethoscopic apparatus as claimed in Claim 9, wherein said signal amplifying means (14) comprises a tact switch (19) which allows the disconnection of said transducers (23, 23') from said signal amplifying means (14) when auscultation has been accomplished or when it is desired to temporarily stop auscultation.

EP 0 454 931 A1

FIG. 1
PRIOR ART

8

FIG.2

F I G. 3

F I G. 4

F I G. 5

F I G. 6

FIG.7

EP 0 454 931 A1

FIG. 8A

FIG.8B

FIG. 8

FIG.9

F I G.10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-3 182 129   (W.B.M. CLARK et al.)<br>* column 3, line 57 - column 6, line 48; figures 1,2 *<br>– – – | 1-4,9 | A 61 B 7/04 |
| A | DE-A-3 638 143   (M.-J. SHUE)<br>* column 7, line 39 - column 8, line 33; figures 4,5 *<br>– – – | 1,5 | |
| A | US-A-4 783 813   (C.W. KEMPKA et al.)<br>* column 2, line 42 - column 4, line 63; figures 1-4 *<br>– – – – – | 1,9 | |

|  |
|---|
| TECHNICAL FIELDS SEARCHED (Int. Cl.5) |
| A 61 B 7/00 |

The present search report has been drawn up for all claims

| Place of search | Date of completion of search | Examiner |
|---|---|---|
| Berlin | 18 December 90 | WEIHS J.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document